# EUROPEAN PATENT APPLICATION

(11) **EP 4 353 735 A1**
(43) Date of publication of application: **17.04.2024**
(21) Application number: 22820270.1
(22) Date of filing: 08.06.2022
(51) Int. Cl.: C07K 1/22, B01D 15/00, B01J 20/28

(54) **SYNTHETIC ADSORBENT, ANTIBODY PURIFICATION METHOD, AND ANTIBODY PRODUCTION METHOD**

(30) Priority: 10.06.2021 JP 2021097149
(71) Applicant: Mitsubishi Chemical Corporation, Chiyoda-ku Tokyo 100-8251 (JP); Kyowa Kirin Co., Ltd., Tokyo 100-0004 (JP)
(72) Inventor: TASHIRO, Yoshiya, Tokyo 100-8251 (JP); OHARA, Shouhei, Tokyo 100-8251 (JP); TAKEHARA, Jun, Tokyo 100-8251 (JP); ISHIHARA, Takashi, Tokyo 100-0004 (JP); KIKUCHI, Shinsuke, Tokyo 100-0004 (JP)
(74) Representative: Ter Meer Steinmeister & Partner
(86) International application number: PCT/JP2022/023149
(87) International publication number: WO 2022/260091

(57) **Abstract**

A synthetic adsorbent,
wherein a maximum value of a differential pore volume (mL/g) under a pressure condition of 0.5 psia to 30.0 psia, which is measured by the following method, is greater than 0.05 mL/g,
<method of measuring differential pore volume (mL/g)>
1. a pressure in a sample container where the synthetic adsorbent that has been dried is placed is reduced to 10 Pa or less, mercury specified in JIS K 8572 is degassed by reducing the pressure to 10 Pa or less, and the sample container is filled with the mercury at a pressure of 0.5 psia,
2. a mercury intrusion amount when the pressure in the sample container filled with the mercury is stepwisely increased from 0.5 psia to 30.0 psia is measured, and
3. a differential pore volume (mL/g) is determined by dividing an amount of an increase in the mercury intrusion amount when a first-stage pressure calculated based on the mercury intrusion amount measured in the item 2 is increased, by a measured amount of the synthetic adsorbent.

## Description

### Technical Field

The present invention relates to a synthetic adsorbent, an antibody purification method, and an antibody production method.

### Background Art

In recent years, biopharmaceuticals are rapidly progressing due to the development of gene recombination technologies. Among biopharmaceuticals, antibody pharmaceuticals are pharmaceuticals in which the production amount has been increasing every year. The antibody pharmaceuticals are extremely expensive pharmaceuticals as compared with other low-molecular-weight pharmaceuticals. One of the reasons why the antibody pharmaceuticals are expensive is that the antibody purification cost is extremely high.

Proteins such as antibodies are typically produced by culturing recombinant cells into which a vector containing genes encoding target proteins is inserted. The culture solution contains, in addition to target proteins, impurities such as a wide variety of culture medium-derived components, host cell-derived components, and protein-derived byproducts. Therefore, it is necessary to separate and remove the impurities so that the pharmaceuticals have a required purity and to purify target proteins.

When an antibody is degraded with proteolytic enzyme papain, the antibody is cleaved at specific locations and divided into parts referred to as an Fab region and an Fc region. Protein A is an example of a protein that can specifically bind to the Fc region. Affinity chromatography using Protein A is a method of adsorbing only an antibody using specificity of this protein to separate the antibody from impurities. This separation method enables only the target antibody to be selectively captured from a culture solution containing a wide variety of impurities. Therefore, this method has been used for purification of most antibody pharmaceuticals.

PTL 1 discloses an antibody purification method performed using affinity chromatography. Further, PTL 2 discloses a purification method performed using activated carbon without using affinity chromatography.

### Citation List

### Patent Literature

PTL 1: Japanese Unexamined Patent Application Publication (Translation of PCT Application) No. H5-504579
PTL 2: WO2014/024514A1

An affinity separation agent of the affinity chromatography using Protein A or the like, disclosed in PTL 1, is extremely expensive as compared with typical separation agents using ion exchange effects or hydrophobic interactions. Therefore, antibody pharmaceuticals produced by using a large amount of the affinity separation agent are also expensive.

As disclosed in PTL 2, the production cost of antibodies can be reduced by a purification method of applying activated carbon without using affinity chromatography. However, the recovery rate of target antibodies is significantly lower than that of affinity chromatography when this method is used.

As described above, it is difficult to realize high production at a low cost by using an antibody purification method of the related art.

### Summary of Invention

### Technical Problem

An object of the present invention is to provide a synthetic adsorbent that reduces the production cost of antibodies and enables efficient antibody production on an industrial scale. Further, an object of the present invention is to provide an antibody purification method and an antibody production method that reduce the production cost of antibodies and enable efficient antibody production on an industrial scale.

The present inventors found that an antibody purification method enabling high production at a low cost can be realized by using a synthetic adsorbent, in which the maximum value of the differential pore volume (mL/g) is greater than a predetermined value, for purification of antibodies, thereby completing the present invention.

That is, the gist of the present invention is as follows.

[1] A synthetic adsorbent,
   wherein a maximum value of a differential pore volume (mL/g) under a pressure condition of 0.5 psia to 30.0 psia, which is measured by the following method, is greater than 0.05 mL/g,
   <method of measuring differential pore volume (mL/g)>
   1. a pressure in a sample container where the synthetic adsorbent that has been dried is placed is reduced to 10 Pa or less, mercury specified in JIS K 8572 is degassed by reducing the pressure to 10 Pa or less, and the sample container is filled with the mercury at a pressure of 0.5 psia,
   2. a mercury intrusion amount when the pressure in the sample container filled with the mercury is stepwisely increased from 0.5 psia to 30.0 psia is measured, and
   3. a differential pore volume (mL/g) is determined by dividing an amount of an increase in the mercury intrusion amount when a first-stage pressure calculated based on the mercury intrusion amount measured in the item 2 is increased, by a measured amount of the synthetic adsorbent.
[2] The synthetic adsorbent according to [1],
   wherein the synthetic adsorbent is a synthetic adsorbent for purifying an antibody.
[3] The synthetic adsorbent according to [1] or [2],
   wherein the synthetic adsorbent has pores.
[4] The synthetic adsorbent according to [3],
   wherein the pores have a radius of 3 nm to 15 nm.
[5] The synthetic adsorbent according to any one of [1] to [4],
   wherein the synthetic adsorbent is hydrophobic.
[6] The synthetic adsorbent according to any one of [1] to [5],
   wherein the synthetic adsorbent contains at least one selected from a styrene-based resin or an acrylic resin.
[7] The synthetic adsorbent according to any one of [1] to [6],
   wherein the synthetic adsorbent has a volume average particle diameter of 1 um to 500 um.
[8] The synthetic adsorbent according to any one of [1] to [7],
   wherein a corrected roundness value determined by the following method is greater than 0.10,
   <method of measuring and calculating corrected roundness value>
   1. the synthetic adsorbent is imaged by an optical microscope,
   2. an approximate circle of the imaged synthetic adsorbent is created using image analysis software WinROOF2018,
   3. the approximate circle is sandwiched between two concentric geometric circles (two concentric circles) that are concentric with the approximate circle in conformity with JIS B 0621,
   4. a difference between a radius of the circle on an outer peripheral side and a radius of the circle on an inner peripheral side when a distance between the two concentric circles in the item 3 is the minimum is determined as the roundness,
   5. the determined roundness is divided by a radius of the approximate circle, and
   6. measurement and calculation in the items 1 to 5 are performed on 100 or more of circles, and an average value thereof is defined as the corrected roundness.
[9] The synthetic adsorbent according to any one of [1] to [8],
   wherein a crushing strength is in a range of 100 gf/particles to 2000 gf/particles.
[10] The synthetic adsorbent according to any one of [2] to [9],
   wherein the antibody is a monoclonal antibody.
[11] The synthetic adsorbent according to [10], wherein the monoclonal antibody has a molecular weight of 100000 or greater.
[12] The synthetic adsorbent according to [10] or [11],
   wherein the monoclonal antibody is immunoglobulin G.
[13] An antibody purification method comprising:
   mixing a mixture that contains antibodies and impurities with the synthetic adsorbent according to any one of [1] to [12] and filtering the mixture.
[14] An antibody purification method comprising:
   a step of causing a mixture that contains antibodies and impurities to pass through the synthetic adsorbent according to any one of [1] to [12] and recovering non-adsorbed fractions containing antibodies, which have not been adsorbed on the synthetic adsorbent.
[15] An antibody purification method comprising:
   a step (i) which is a contact step of brining a mixed solution that contains antibodies and impurities into contact with the synthetic adsorbent according to any one of [1] to [12]; and
   a step (ii) which is a separation step of separating the mixed solution and the synthetic adsorbent from each other after the step (i).
[16] The antibody purification method according to [15], further comprising:
   a step (A) which is an ion exchange resin contact step of bringing a mixed solution that contains antibodies and impurities into contact with an anion exchange resin and/or a cation exchange resin,
   wherein the step (A) is performed before the step (i).
[17] The antibody purification method according to any one of [13] to [16],
   wherein the impurities include a substance having a molecular weight of 50000 or less.
[18] The antibody purification method according to any one of [13] to [17],
   wherein the impurities include at least one selected from the group consisting of host cell-derived proteins, antibody-derived polymers, antibody-derived degradation products, and nucleic acids.
[19] The antibody purification method according to any one of [13] to [18],
   wherein the mixing of the mixture with the synthetic adsorbent, the passing of the mixture through the synthetic adsorbent, or the contacting of the mixed solution with the synthetic adsorbent are performed in a liquid having a pH of 3 to 8.
[20] An antibody production method comprising:
   the antibody purification method according to any one of [13] to [19].

Further, the second gist of the present invention is as follows.
<1> A synthetic adsorbent which has been crushed for antibody purification.
<2> The synthetic adsorbent according to <1>, in which the synthetic adsorbent has pores.
<3> The synthetic adsorbent according to <2>, wherein the pores have a radius of 3 nm to 15 nm.
<4> The synthetic adsorbent according to any one of <1> to <3>,
   wherein the synthetic adsorbent is hydrophobic.
<5> The synthetic adsorbent according to any one of <1> to <4>,
   wherein the synthetic adsorbent contains at least one selected from a styrene-based resin or an acrylic resin.
<6> The synthetic adsorbent according to any one of <1> to <5>,
   wherein the synthetic adsorbent has a volume average particle diameter of 1 um to 500 um.
<7> The synthetic adsorbent according to any one of <1> to <6>,
   wherein the antibody is a monoclonal antibody.
<8> The synthetic adsorbent according to <7>,
   wherein the monoclonal antibody has a molecular weight of 100000 or greater.
<9> The synthetic adsorbent according to <7> or <8>,
   wherein the monoclonal antibody is immunoglobulin G.
<10> An antibody purification method comprising:
   mixing a mixture that contains antibodies and impurities with the synthetic adsorbent according to any one of <1> to <9> and filtering the mixture.
<11> An antibody purification method comprising:
   a step of causing a mixture that contains antibodies and impurities to pass through the synthetic adsorbent according to any one of <1> to <9> and recovering non-adsorbed fractions containing antibodies, which have not been adsorbed on the synthetic adsorbent.
<12> The antibody purification method according to any one of <10> to <11>,
   wherein the impurities include a substance having a molecular weight of 50000 or less.
<13> The antibody purification method according to any one of <10> to <12>,
   wherein the impurities include at least one selected from the group consisting of host cell-derived proteins, antibody-derived polymers, antibody-derived degradation products, and nucleic acids.
<14> The antibody purification method according to any one of <10> to <13>,
   wherein the mixing of the mixture with the synthetic adsorbent is performed in a liquid having a pH of 3 to 8.
<15> An antibody production method comprising:
   the antibody purification method according to any one of <10> to <14>.

### Advantageous Effects of Invention

The synthetic adsorbent of the present invention enables purification of antibodies, reduction of the production cost, and efficient production of antibodies on an industrial scale.

The antibody purification method and the antibody production method of the present invention enable purification of antibodies, reduction of the production cost, and efficient production of antibodies on an industrial scale.

### Brief Description of Drawing

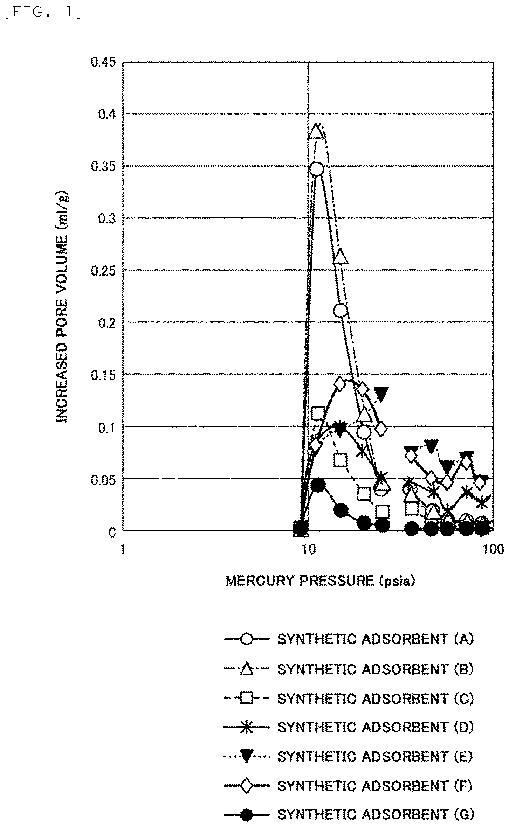

[Fig. 1] Fig. 1 is a graph showing measured values of differential pore volumes (mL/g) of synthetic adsorbents (A) to (G) in examples and comparative examples.

### Description of Embodiments

Hereinafter, the present invention will be described in detail. The present invention is not limited to the following embodiments and various modifications can be made within the range of the gist of the present invention.

In the present specification, when a range of numerical values is shown using "-", the range includes the numerical values or physical property values before and after "

In the present specification, "(meth)acryl" denotes any one or both "acryl" and "methacryl". "(Meth)acrylate" denotes any one or both "acrylate" and "methacrylate". The same applies to "(meth)acryloyl".

### (Synthetic adsorbent)

A synthetic adsorbent of the present invention is a synthetic adsorbent in which the maximum value of the differential pore volume (mL/g) under a pressure condition of 0.5 psia to 30.0 psia, which is measured by the following method, is greater than 0.05 mL/g.

### <method of measuring differential pore volume (mL/g)>

1. a pressure in a sample container where the synthetic adsorbent that has been dried is placed is reduced to 10 Pa or less, mercury specified in JIS K 8572 is degassed by reducing the pressure to 10 Pa or less, and the sample container is filled with the mercury at a pressure of 0.5 psia,
2. a mercury intrusion amount when the pressure in the sample container filled with the mercury is stepwisely increased from 0.5 psia to 30.0 psia is measured, and
3. a differential pore volume (mL/g) is determined by dividing an amount of an increase in the mercury intrusion amount when a first-stage pressure calculated based on the mercury intrusion amount measured in the item 2 is increased, by a measured amount of the synthetic adsorbent.

The mercury porosimetry is a method of measuring the void or pore volume, which is measured by pressing mercury under pressure using the characteristic that mercury has a high surface tension and thus does not enter void or pores only with a force of the atmospheric pressure. The measurement is performed by stepwisely increasing the pressure, detecting the amount of injected mercury using a detector such as a capacitance type detector, and calculating the pore volume using the amount of the increased mercury injected.

A value obtained by stepwisely increasing the pressure to measure the mercury intrusion amount and dividing the amount of an increase in the mercury intrusion amount when a first-stage pressure calculated based on the measured mercury intrusion amount is increased, by the measured amount of the synthetic adsorbent is defined as the differential pore volume (mL/g).

In the measurement of the differential pore volume (mL/g), the range of an increase in pressure for each measurement when the pressure is stepwisely increased depends on the specification of a pore volume measuring device to be used, but is preferably in a range of 2 psia to 10 psia and particularly preferably in a range of 2 psia to 5 psia from the viewpoint that the differential pore volume (mL/g) according to the present invention can be accurately determined.

In the present invention, as specifically described in the examples below, the differential pore volume (mL/g) is determined by the mercury porosimetry from an increase in the mercury intrusion amount when the measured pressure is stepwisely increased using a pore distribution measuring device (model name "AUTOPORE IV9520", manufactured by Micromeritics Instruments Corporation). It is preferable that the measured pressure value of the mercury intrusion amount as an example be defined as the following pressure values.
First measured pressure value: 9.0 ± 0.5 psia
Second measured pressure value: 11.0 ± 0.5 psia
Third measured pressure value: 15.0 ± 0.5 psia
Fourth measured pressure value: 20.0 ± 0.5 psia
Fifth measured pressure value: 25.0 ± 0.5 psia

In the synthetic adsorbent of the present invention, the maximum value of the differential pore volume (mL/g) at a measured pressure of 0.5 psia to 30.0 psia is greater than 0.05 mL/g. The synthetic adsorbent in which the maximum value of the differential pore volume (mL/g) is greater than 0.05 mL/g has pores with excellent impurity removability in the particle surfaces of the synthetic adsorbent and has excellent impurity removability and excellent antibody purification efficiency. From the viewpoint of excellent impurity removability, the maximum value of the differential pore volume (mL/g) of the synthetic adsorbent according to the present invention is greater than 0.05 mL/g, preferably 0.1 mL/g or greater, more preferably 0.2 mL/g or greater, and still more preferably 0.3 mL/g or greater. Meanwhile, the upper limit of the maximum value of the differential pore volume (mL/g) is not particularly limited, but is typically 1.0 mL/g or less from the viewpoints of accuracy of measurement and the physical strength of the synthetic adsorbent.

The synthetic adsorbent in which the maximum value of the differential pore volume (mL/g) is greater than 0.05 mL/g can be obtained, for example, by crushing particles of a commercially available synthetic adsorbent (synthetic resin) or particles of a produced synthetic adsorbent (synthetic resin) such that the synthetic adsorbent satisfying the maximum value of the differential pore volume (mL/g) is obtained, as described below.

The synthetic adsorbent denotes a synthetic substance that optimizes the matrix structure, the pore structure, the surface area, the polarity of the surface, and the like and adsorbs a specific organic compound from a solution using a physical interaction or a chemical interaction.

In the present invention, the synthetic adsorbent denotes an organic synthetic adsorbent.

Examples of the material of the synthetic adsorbent include a styrene-based resin, an acrylic resin, a phenolic resin, and an amide-based resin. These materials of the synthetic adsorbent may be used alone or in combination of two or more kinds thereof. Among these materials of the synthetic adsorbent, from the viewpoint that the cost is low and the hydrophobicity is excellent, a styrene-based resin and an acrylic resin are preferable, and a styrene-based resin is more preferable.

The hydrophobicity denotes a property that non-polar groups with a low affinity for water molecules are likely to be collected in an aqueous solution. The interaction using the attractive force denotes a hydrophobic interaction.

Examples of the hydrophobic functional group include functional groups of hydrocarbons such as an alkyl group, a phenyl group, and a benzyl group.

In the present specification, the styrene-based resin is a resin in which the content of a constitutional unit derived from an aromatic vinyl monomer in 100% by mass of all monomer units constituting the styrene-based resin is 50% by mass or greater. From the viewpoint that the adsorption amount of impurities to the synthetic adsorbent, the physical strength of the synthetic adsorbent, and the formability of pores are excellent, the proportion of the styrene-based resin is preferably 80% by mass or greater. The styrene-based resin may have other constitutional units in addition to the constitutional unit derived from an aromatic vinyl monomer.

Examples of the aromatic vinyl monomer include aromatic monovinyl monomers such as styrene, methylstyrene, ethylstyrene, α-methylstyrene, chlorostyrene, chloromethylstyrene, and bromobutylstyrene, and crosslinkable aromatic vinyl monomers such as divinylbenzene, bis(vinylphenyl)ethane, divinylnaphthalene, and 2,4,6-trivinylethylbenzene. These aromatic vinyl monomers may be used alone or in combination of two or more kinds thereof.

Among these aromatic vinyl monomers, from the viewpoint that the adsorption amount of impurities to the synthetic adsorbent, the physical strength of the synthetic adsorbent, and the formability of pores are excellent, the styrene-based resin has preferably a crosslinkable aromatic vinyl monomer, more preferably a combination of an aromatic monovinyl monomer and a crosslinkable aromatic vinyl monomer, and still more preferably a combination of styrene and divinylbenzene.

The content of the constitutional unit derived from the aromatic monovinyl monomer in the styrene-based resin is preferably in a range of 0% by mass to 99% by mass and more preferably in a range of 20% by mass to 80% by mass with respect to 100% by mass of all monomer units constituting the styrene-based resin. When the content of the constitutional unit derived from the aromatic monovinyl monomer is greater than or equal to the above-described lower limits, the flexibility of the synthetic adsorbent is excellent. When the content of the constitutional unit derived from the aromatic monovinyl monomer is less than or equal to the above-described upper limits, dissolution of the synthetic adsorbent in a solvent, and swelling or contraction of the synthetic adsorbent can be suppressed.

The content of the constitutional unit derived from the crosslinkable aromatic vinyl monomer in the styrene-based resin is preferably in a range of 1% by mass to 100% by mass and more preferably in a range of 20% by mass to 80% by mass with respect to 100% by mass of all monomer units constituting the styrene-based resin. When the content of the constitutional unit derived from the crosslinkable aromatic vinyl monomer is greater than or equal to the above-described lower limits, dissolution of the synthetic adsorbent in a solvent, and swelling or contraction of the synthetic adsorbent can be suppressed. When the content of the constitutional unit derived from the crosslinkable aromatic vinyl monomer is less than or equal to the above-described upper limits, the flexibility of the synthetic adsorbent is excellent.

From the viewpoint that the synthetic adsorbent has excellent hydrophobicity, the content of the constitutional unit derived from other monomers in addition to the aromatic monovinyl monomer unit and the crosslinkable aromatic vinyl monomer unit in the styrene-based resin is preferably 10% by mass or less, more preferably 1% by mass or less, and still more preferably 0% by mass with respect to 100% by mass of all monomer units constituting the styrene-based resin.

In the present specification, the content of the constitutional unit derived from a (meth)acrylate in the acrylic resin is 50% by mass or greater with respect to 100% by mass of all monomer units constituting the acrylic resin. From the viewpoint that the recovery rate of antibodies is excellent, the proportion thereof is preferably 80% by mass or greater. The acrylic resin may have constitutional units other than the constitutional unit derived from a (meth)acrylate.

Examples of (meth)acrylates include alkyl (meth)acrylates such as methyl (meth)acrylate, ethyl (meth)acrylate, butyl (meth)acrylate, stearyl (meth)acrylate, 2-ethylhexyl (meth)acrylate, and cyclohexyl (meth)acrylate; hydroxyl group-containing (meth) acrylates such as hydroxyethyl (meth) acrylate, hydroxypropyl (meth) acrylate, glycerin mono (meth) acrylate; epoxy group-containing (meth)acrylates such as glycidyl (meth) acrylate, 4,5-epoxybutyl (meth) acrylate and 9,10-epoxystearyl (meth)acrylate; (meth)acrylamides such as (meth)acrylamide, dimethyl (meth)acrylamide and hydroxyethyl (meth)acrylamide; cyano group-containing (meth)acrylates such as (meth)acrylonitrile; alkylene di (meth) acrylates such as ethylene glycol di (meth) acrylate; polyalkylene glycol di (meth) acrylates such as polyethylene glycol di (meth) acrylate; crosslinkable (meth)acrylates such as N,N'-alkylene bis(meth)acrylamide, glycerol di(meth)acrylate, trimethylolpropane tri(meth)acrylate, and the like. These (meth)acrylates may be used alone or in combination of two or more kinds thereof.

Among these (meth)acrylates, from the viewpoint that the synthetic adsorbent is easily functionalized and the physical strength of the synthetic adsorbent is excellent, the acrylic resin contains preferably crosslinkable (meth)acrylate and more preferably ethylene glycol di(meth)acrylate.

The content of the crosslinkable (meth)acrylate unit in the acrylic resin is preferably in a range of 1% by mass to 100% by mass and more preferably in a range of 20% by mass to 80% by mass with respect to 100% by mass of all monomer units constituting the acrylic resin. When the content of the crosslinkable (meth)acrylate unit is greater than or equal to the above-described lower limits, dissolution of the synthetic adsorbent in a solvent, and swelling or contraction of the synthetic adsorbent can be suppressed. When the content of the crosslinkable (meth)acrylate unit is less than or equal to the above-described upper limits, the flexibility of the synthetic adsorbent is excellent.

The synthetic adsorbent of the present invention can be obtained by crushing synthetic resin particles or a commercially available product, produced by a known polymerization method such as suspension polymerization or emulsion polymerization.

The crushing denotes that the synthetic adsorbent is finely grounded by a physical force.

The instrument used for crushing is not particularly limited as long as the synthetic adsorbent can be finely grounded by a physical force, and examples thereof include a mortar, a mixer, a ball mill, a hammer mill, and a pin mill. Among these instruments used for crushing, from the viewpoint of finely crushing the synthetic adsorbent and easily controlling the particle size distribution, a ball mill is preferable.

From the viewpoint that the specific surface area is efficiently increased and the impurity removability is excellent, it is preferable that the synthetic adsorbent of the present invention have pores.

The pores of the synthetic adsorbent may be formed by using a porosifier during polymerization in a case of obtaining a synthetic resin.

The radius of pores of the synthetic adsorbent is preferably in a range of 3 nm to 15 nm and more preferably in a range of 5 nm to 10 nm. When the radius of pores of the synthetic adsorbent is greater than or equal to the above-described lower limits, the diffusibility of impurities in the synthetic adsorbent is excellent, and the impurity removability is excellent. When the radius of pores of the synthetic adsorbent is less than or equal to the above-described upper limits, the adsorption amount of impurities to the synthetic adsorbent is excellent, a decrease in the yield due to the diffusion of antibodies in the synthetic adsorbent can be suppressed. That is, the recovery rate of antibodies during the antibody purification and the productivity of antibodies are excellent.

The radius of pores of the synthetic adsorbent is defined as the most frequent radius of pores measured using a pore distribution measuring device by applying nitrogen gas.

The radius of pores of the synthetic adsorbent can be adjusted by controlling the kind or the addition amount of the porosifier and the polymerization rate in a case of obtaining a synthetic resin.

The volume average particle diameter of the synthetic adsorbent is preferably in a range of 1 um to 500 um and more preferably in a range of 10 um to 200 um. When the volume average particle diameter of the synthetic adsorbent is greater than or equal to the above-described lower limits, the filterability during the antibody purification is excellent, and the recovery rate of antibodies and the productivity of antibodies are excellent. When the volume average particle diameter of the synthetic adsorbent is less than or equal to the above-described upper limits, the surface area of the synthetic adsorbent can be increased, and the performance of removing the impurities is excellent.

The volume average particle diameter of the synthetic adsorbent is defined as a value measured using a laser diffraction scattering particle size analyzer.

A measurement sample obtained by immersing the synthetic adsorbent in a 20 vol% ethanol aqueous solution and removing adhesive moisture by filtration is used as the measurement sample.

The corrected roundness value of the synthetic adsorbent according to the present invention is preferably greater than 0.10 and more preferably 0.40 or greater. When the corrected roundness value is greater than 0.10, the diffusibility of the synthetic adsorbent inside the pores is high, and the performance of removing the impurities is excellent. The upper limit of the corrected roundness is not particularly limited, but is typically 1.00 or less.

Here, the term "roundness" is defined in JIS B 0621 and denotes "amount of deviation of a circular shape from a geometrically correct circle". The roundness denotes "difference between radii of two circles when the distance between two concentric circles is the minimum in a case where an object (circular shape) is sandwiched between two concentric geometric circles".

According to the present invention, "corrected roundness" is obtained by dividing this roundness by the radius of the approximate circle of the synthetic adsorbent imaged when the roundness is determined and corresponds to a value obtained by correcting a change of the roundness value due to the particle diameter. The circular shape is closer to a geometric circle as the corrected roundness is closer to 0, and the circular shape is more deviated from a geometric circle as the corrected roundness increases.

The corrected roundness according to the present invention is determined by the following method. Further, the length units of the radius values of the roundness and the approximate circle described below are set to be the same as each other.

### <method of measuring and calculating corrected roundness value>

1. the synthetic adsorbent is imaged by an optical microscope,
2. an approximate circle of the imaged synthetic adsorbent is created using image analysis software WinROOF2018,
3. the approximate circle is sandwiched between two concentric geometric circles (two concentric circles) that are concentric with the approximate circle in conformity with JIS B 0621,
4. a difference between a radius of the circle on an outer peripheral side and a radius of the circle on an inner peripheral side when a distance between the two concentric circles in the item 3 is the minimum is determined as the roundness,
5. the determined roundness is divided by a radius of the approximate circle, and
6. measurement and calculation in the items 1 to 5 are performed on 100 or more of circles, and an average value thereof is defined as the corrected roundness.

The crushing strength (physical strength) of the synthetic adsorbent is preferably in a range of 100 gf/particles to 2000 gf/particles and more preferably in a range of 300 gf/particles to 1000 gf/particles. Typically, it is preferable that the crushing strength is high from the viewpoint that the synthetic adsorbent is unlikely to be crushed when used. However, the synthetic adsorbent with an extremely high crushing strength cannot withstand swelling and contraction due to a change in the composition of a solvent that passes through the synthetic adsorbent and thus is crushed. From the viewpoint of the balance between the resistance to a physical impact such as a load due to lamination of the particles of the synthetic adsorbent or collision due to stirring and the resistance to swelling and contraction due to a change in composition of a solvent, it is preferable that the crushing strength is in the above-described ranges.

Here, the crushing strength is an average value of forces preventing an object from maintaining the original shape (forces destroying the original shape) when the object receives the crushing (compression) force. The crushing strength is measured, specifically, by the method described in the examples below.

In the examples below, the measurement of the crushing strength is performed on the synthetic adsorbent before being crushed. The reason for this is that the particles of the crushed synthetic adsorbent are small and thus measurement results cannot be obtained. Since the crushing strength is the strength of a material, generally in a case of the same composition, the strength is high when the composition is small. Therefore, it is considered that the value does not decrease after crushing, and thus the measured value of the crushing strength of the synthetic adsorbent before crushing is greater than or equal to the measured value of the synthetic adsorbent after crushing.

### (Antibody)

It is preferable that the synthetic adsorbent of the present invention is used for purification of antibodies.

An antibody is a protein produced in a living body due to an immune reaction caused as a result of antigen stimulation and has the activity of specifically binding to an antigen.

Examples of the antibody include mouse antibodies, llama antibodies, chimeric antibodies, humanized antibodies, human antibodies, antibodies obtained by modifying Fc regions or the like of these antibodies, multivalent antibodies of these antibodies, and antibodies obtained by modifying a part of these antibodies. Examples of the molecular type of antibodies include immunoglobulin G (IgG), immunoglobulin M (IgM), immunoglobulin A (IgA), immunoglobulin D (IgD), immunoglobulin E (IgE), Fab, Fe, Fc-fusion proteins, VH, VL, VHH, Fab'2, scFv, scFab, scDb, and scDbFc. Among these antibodies, from the viewpoint of excellent simplicity, antibodies derived from the same cell and antibodies of the same class are preferable, and the monoclonal antibodies are more preferable.

The monoclonal antibody is an antibody produced by a single clone of antibody forming cells and has a uniform primary structure, that is, a uniform amino acid sequence. Accordingly, the class, the subclass, the allotype, the L chain type, and the like are uniform.

Examples of the monoclonal antibody include immunoglobulin G (IgG), immunoglobulin M (IgM), immunoglobulin A (IgA), immunoglobulin D (IgD), and immunoglobulin E (IgE). Among these monoclonal antibodies, from the viewpoint that the antibody is unlikely to be adsorbed on the synthetic adsorbent, IgG, IgD, and IgE are preferable, and IgG is more preferable.

The molecular weight of the monoclonal antibody is preferably in a range of 100000 to 9000000 and more preferably in a range of 300000 to 2000000. The molecular weight of the monoclonal antibody is greater than or equal to the above-described lower limits, a decrease in yield due to the diffusion of the antibody inside the synthetic adsorbent can be suppressed. Further, when the molecular weight of the monoclonal antibody is less than or equal to the above-described upper limits, the monoclonal antibody is unlikely to be precipitated in water and easily dissolved therein.

The molecular weight of the monoclonal antibody is a value measured by size exclusion chromatography using a molecular sieve effect.

### (Antibody purification method)

A method of purifying antibodies using the synthetic adsorbent of the present invention is not particularly limited, and examples thereof include the following methods (1) to (3).

(1) An antibody purification method including mixing a mixture that contains antibodies and impurities with the synthetic adsorbent of the present invention and filtering the mixture. That is, the synthetic adsorbent to which impurities are adsorbed and the antibodies dissolved in a liquid are separated by filtration.
(2) A so-called flow-through type purification method including a step of causing a mixture that contains antibodies and impurities to pass through the synthetic adsorbent of the present invention and recovering non-adsorbed fractions containing antibodies, which have not been adsorbed on the synthetic adsorbent.
(3) An antibody purification method including the following step (A) before the following steps (i) and (ii).
   Step (i): a contact step of brining a mixed solution that contains antibodies and impurities into contact with the synthetic adsorbent of the present invention; and
   Step (ii): a separation step of separating the mixed solution and the synthetic adsorbent from each other after the step (i)
   Step (A): an ion exchange resin contact step of bringing a mixed solution that contains antibodies and impurities into contact with an anion exchange resin and/or a cation exchange resin

A desalting treatment carried out by the step (A) can increase the impurity removal efficiency, the antibody purification efficiency, and the antibody recovery efficiency due to the synthetic adsorbent of the present invention. Further, the mixture of the anion exchange resin and/or the cation exchange resin used in the step (A) may be mixed with the synthetic adsorbent used in the step (i). The step (i) may be a batch method or a flow-through method, but the step (ii) can be omitted when the step (i) is a batch method.

The impurities are required to be removed in order to use antibodies as pharmaceutical products, and examples thereof include culture medium components necessary for culturing recombinant cells, proteins and metabolites other than cell-derived antibodies, and nucleic acids.

Specifically, the impurities include at least one selected from the group consisting of host cell-derived proteins, antibody-derived polymers, antibody-derived degradation products, and nucleic acids.

The host cell-derived proteins are proteins produced in the growing process in a case of culturing antibody-producing cells, cell debris of dead cells, and the like, and examples thereof include modified products derived from proteins that have undergone removal of sugar chain components, oxidation, deamidation, or the like, and enzymes eluted from host cells.

Examples of the enzymes eluted from host cells include sugar-removing enzymes, protein hydrolases, oxidoreductases, and amino acid isomerases.

Examples of the sugar-removing enzymes include neuraminidase (sialidases), galactosidases, and glycanases.

Examples of the protein hydrolases include serine proteases, esterases, cysteine proteases, trypsin-like proteases, aminopeptidases, aspartic proteases, and cathepsins.

Examples of the oxidoreductases include thioredoxin-related enzymes such as thioredoxin reductases.

The antibody-derived polymers are obtained by polymerizing antibodies using heat, catalysts, pH conditions, and the like, and examples thereof include heat-denatured polymers of human γ-globulin and polymers produced by oxidation.

The antibody-derived degradation products are fragments in which a peptide bond or a disulfide bond of an antibody is cleaved and degraded due to protein hydrolases, heat, pH conditions, and the like.

The nucleic acids are long-chain polynucleotides containing nucleotides formed of purine, a pyrimidine base, pentose, and phosphoric acid as a base unit, in which phosphoric acid is polymerized by a diester bond between the 3' carbon and the 5' carbon of sugar between nucleotides, and examples thereof include DNA, RNA, and degradation products thereof.

The molecular weight of the impurities is preferably in a range of 1000 to 50000 and more preferably in a range of 5000 to 25000. When the molecular weight of the impurities is greater than or equal to the above-described lower limits, the impurities have hydrophobic portions and are likely to be desorbed to the synthetic adsorbent. Further, when the molecular weight of the impurities is less than or equal to the above-described upper limits, the diffusibility of the impurities inside the synthetic adsorbent is excellent.

The molecular weight of the impurities is a value measured by size exclusion chromatography using a molecular sieve effect.

The filtration in the method (1) described above is performed by separating a solid from a liquid using a filter material.

Examples of the filter material include a hydrophilic sulfone-based polymer membrane, a hydrophilic aromatic ether-based polymer membrane, a hydrophilic fluorine-based polymer membrane, a hydrophilic olefin-based polymer membrane, a cellulose-based membrane, a (meth)acrylic polymer membrane, a (meth)acrylonitrile-based polymer membrane, and a vinyl alcohol-based polymer membrane. Among these filter materials, from the viewpoint of excellent hydrophilicity, a hydrophilic fluorine-based polymer membrane, a hydrophilic sulfone-based polymer membrane, and a cellulose-based membrane are preferable, and a hydrophilic fluorine-based polymer membrane is more preferable.

The pore diameter of the filter material is preferably in a range of 0.1 um to 30 um and more preferably in a range of 0.2 um to 10 um. When the pore diameter of the filter material is greater than or equal to the above-described lower limits, liquid permeability of the filtration is excellent. Further, when the pore diameter of the filter material is less than or equal to the above-described upper limits, the separability of the solid from the liquid is excellent.

The pore diameter of the filter material is measured using an image obtained with a scanning electron microscope (SEM).

Examples of the method of bringing the mixed solution that contains antibodies and impurities into contact with the synthetic adsorbent of the present invention in the step (i) of the method (3) include a method of mixing the mixed solution that contains antibodies and impurities with the synthetic adsorbent of the present invention. Further, as in the method (2), a flow-through type method of causing the mixed solution to pass through a column filled with the synthetic adsorbent of the present invention may be employed.

Examples of the anion exchange resin used in the step (A) of the method (3) include a basic anion exchange resin which contains an exchange group such as a quaternary ammonium (triethylammonium or trimethylammonium) group in a crosslinked polymer formed of a monovinyl aromatic monomer and a crosslinkable aromatic monomer and is dissociated similarly to strong alkalis such as sodium hydroxide and potassium hydroxide and exhibits basicity. Particularly, an anion exchange resin exhibiting ion exchange properties in the entire pH range (0 to 14) is preferable, and a strongly basic anion exchange resin is preferable. Examples of a commercially available basic anion exchange resin include SA Series, PA Series, and UBA Series of DIAION (registered trademark) (manufactured by Mitsubishi Chemical Corporation). Specific examples of the basic anion exchange resin include a basic anion exchange resin that contains a quaternary ammonium (trimethylammonium or triethylammonium) group as an exchange group and is of a OH type or a Cl type as a salt form. Among these, the OH type is preferable from the viewpoint of desalting efficiency.

Meanwhile, examples of the cation exchange resin include an acidic cation exchange resin which is an ion exchange resin containing an exchange group such as a sulfonic acid group (-SO₃H) in a crosslinked polymer formed of a monovinyl aromatic monomer and a crosslinkable aromatic monomer and is dissociated similarly to mineral acids such as hydrochloric acid and sulfuric acid and exhibits acidity. Particularly, a cation exchange resin exhibiting ion exchange properties in the entire pH range (0 to 14) is preferable, and a strongly acidic cation exchange resin is preferable. Examples of a commercially available acidic cation exchange resin include SK Series, PK Series, and UBK Series of DIAION (registered trademark) (manufactured by Mitsubishi Chemical Corporation). Specific examples of the acidic cation exchange resin include an acidic cation exchange resin that contains a sulfonic acid group as an exchange group and is typically of a H type or a Li type as a salt form. Among these, the H type is preferable from the viewpoint of desalting efficiency.

Further, the ion exchange resin is broadly classified into "gel type" and "porous type" in terms of the structural properties, but it is preferable that both the acidic cation exchange resin and the basic anion exchange resin used in the present invention are of a gel type.

That is, the gel type ion exchange resin has a larger ion exchange capacity per volume and higher physical strength (crushing strength) than those of the porous type ion exchange resin and thus can be used for a long time.

Only one of the anion exchange resin or the cation exchange resin may be used in the desalting treatment of the step (A), but it is preferable that the anion exchange resin and the cation exchange resin may be used by being mixed or combined from the viewpoint of removing both the cationic impurities and anionic impurities.

The desalting treatment of the step (A) can be performed by mixing the mixed solution containing antibodies and impurities with the anion exchange resin and/or the cation exchange resin or causing the mixed solution containing antibodies and impurities to pass through the anion exchange resin floor and/or the cation exchange resin floor, preferably a mixed floor of the anion exchange resin and the cation exchange resin.

In both cases, it is preferable that a treatment liquid obtained by the desalting treatment of the step (A) exhibits a conductivity of 10 mS/cm or less and particularly a low conductivity of 5 mS/cm or less by removal of the ionic impurities from the viewpoint of improving the impurity removal efficiency, the antibody purification efficiency, and the antibody recovery efficiency due to the desalting treatment.

In the methods (1) to (3) described above, the mixing of the mixture with the synthetic adsorbent, the passing of the mixture through the synthetic adsorbent, or the contacting of the mixed solution with the synthetic adsorbent are performed preferably in a liquid having a pH of 3 to 8 and more preferably in a liquid having a pH of 4 to 6. When the pH thereof is greater than or equal to the above-described lower limits, the stability of the antibodies can be maintained. Further, the pH thereof is less than or equal to the above-described upper limits, the adsorption of antibodies to the synthetic adsorbent can be suppressed.

Therefore, acids, alkalis, or the like may be added to the mixture or mixed solution containing antibodies and impurities as necessary for adjusting the pH.

### (Antibody production method)

An antibody production method of the present invention includes the above-described antibody purification method.

The antibody production method of the present invention may include, in addition to the antibody purification step described above, a cell culture step, a purification step using an ion exchange resin, a purification step using size exclusion chromatography, and a purification step using membrane separation.

### (Applications)

The synthetic adsorbent of the present invention reduces the production cost of antibodies and enables efficient antibody production on an industrial scale. Further, the antibody purification method and the antibody production method of the present invention reduce the production cost of antibodies and enable efficient antibody production on an industrial scale.

### EXAMPLES

Hereinafter, the present invention will be described in more detail based on examples, and the present invention is not limited to the description of the examples as long as the description thereof does not depart from the gist of the present invention.

### (Measurement of volume average particle diameter and particle size distribution)

The volume average particle diameters and the particle size distributions of synthetic adsorbents (A) to (G) were measured using a laser diffraction scattering particle size analyzer (model name "MLS-2000e", manufactured by Seishin Enterprise Co., Ltd.).

### (Measurement of differential pore volume)

The differential pore volumes of the synthetic adsorbents (A) to (G) when the pressure was stepwisely increased from 0.5 psia to 30.0 psia were measured by the mercury porosimetry using a pore distribution measuring device (model name "AUTOPORE IV9520", manufactured by Micromeritics Instruments Corporation).

As an example, the actual measured pressures and the measured values of the differential pore volumes of the synthetic adsorbents (A) to (G) are listed in Table 1.

**[Table 1]**

| Synthetic adsorbent (A) | | Synthetic adsorbent (B) | | Synthetic adsorbent (C) | | Synthetic adsorbent (D) | | Synthetic adsorbent (E) | | Synthetic adsorbent (F) | | Synthetic adsorbent (G) | |
|---|---|---|---|---|---|---|---|---|---|---|---|---|---|
| Pressure (psia) | Differential pore volume (mL/g) | Pressure (psia) | Differential pore volume (mL/g) | Pressure (psia) | Differential pore volume (mL/g) | Pressure (psia) | Differential pore volume (mL/g) | Pressure (psia) | Differential pore volume (mL/g) | Pressure (psia) | Differential pore volume (mL/g) | Pressure (psia) | Differential pore volume (mL/g) |
| 8.94995 | 0 | 8.94995 | 0 | 8.94995 | 0 | 8.94995 | 0 | 8.94995 | 0 | 8.94995 | 0 | 8.94995 | 0 |
| 8.9742 | 0.00521 | 8.9742 | 0.00495 | 8.9742 | 0.00156 | 8.9728 | 0.00113 | 8.9728 | 0.000996 | 8.9728 | 0.000888 | 8.97447 | 0.00052 |
| 11.1633 | 0.34807 | 11.1633 | 0.38265 | 11.1633 | 0.11198 | 11.1613 | 0.08695 | 11.1613 | 0.07989 | 11.1613 | 0.08337 | 11.1622 | 0.04394 |
| 14.8842 | 0.21187 | 14.8842 | 0.26188 | 14.8842 | 0.06854 | 14.8764 | 0.10011 | 14.8764 | 0.09784 | 14.8764 | 0.14054 | 14.8829 | 0.02075 |
| 19.8646 | 0.09594 | 19.8646 | 0.10969 | 19.8646 | 0.0357 | 19.8642 | 0.07694 | 19.8642 | 0.1093 | 19.8642 | 0.13576 | 19.8654 | 0.00749 |
| 24.8525 | 0.04183 | 24.8525 | 0.04355 | 24.8525 | 0.01821 | 24.8574 | 0.05143 | 24.8574 | 0.1310 | 24.8574 | 0.09784 | 24.8658 | 0.00576 |

### (Measurement of radius of pores)

The most frequent radii of pores of the synthetic adsorbents (D) to (G) were measured using a pore distribution measuring device (model name "ASAP2024", manufactured by Micromeritics Instruments Corporation) by applying nitrogen gas. In addition, the radii of the pores did not change significantly before and after crushing.

Microphotographs of the synthetic adsorbents (A) to (G) were imaged using Nikon SMA18 (manufactured by Nikon Solutions Co., Ltd.). Each radius of the two concentric circles and the radius of the approximate circle were measured using image analysis software WinROOF2018 (manufactured by Mitani Corporation), and the roundness and the corrected roundness obtained by dividing the roundness by the radius of the approximate circle were calculated.

### (Measurement of crushing strength)

The particles of the synthetic adsorbents (the synthetic adsorbents (A) to (C) and (E) were the same as each other) before crushing, which were used for producing the synthetic adsorbents (A) to (F) were immersed in desalted water and stored in the desalted water until immediately before the measurement. Next, at least 30 particles were randomly selected from the synthetic adsorbent particles before crushing, the strength was measured using Charillon force measurement CS225 (AMETEK TEST & CALIBRATION INSTRUMENTS), and the average value of the strengths of all the particles was calculated. In addition, since the synthetic adsorbent (G) had a small volume particle diameter (um), the crushing strength was difficult to measure.

### (Measurement of concentration of impurities and recovery rate of antibodies)

As a culture solution of monoclonal antibodies (immunoglobulin G, molecular weight: 150000), a culture solution obtained by causing Chinese hamster ovary-derived cells (CHO cells) to produce antibodies, removing the CHO cells, and being clarified was used. The pH of the culture solution was adjusted from 7.4 to 4.5 by using a 5 mol/L acetic acid aqueous solution.

Next, 0.2 g of each of the synthetic adsorbents (A) to (G) was weighed into a 15 mL container, and 4 mL of the prepared culture solution was added to each container. After completion of the addition, the mixture was shaken and mixed for 8 hours. Thereafter, the synthetic adsorbent was removed by performing filtration using a membrane filter (manufactured by Merck & Co., Inc., pore diameter of 0.22 um, PVDF membrane), thereby obtaining a synthetic adsorbent treatment liquid.

The concentration of host cell-derived proteins (HCP concentration [ng/mL] was measured by an ELISA method using a kit (kit name "ELISA Kit F550 (CHO Host Cell Protein 3Rd Generation)").

The logarithmic reduction value (LRV) of the HCP concentration was calculated from the HCP concentrations before and after the treatment.

The recovery rate [%] of the antibodies was measured by high performance liquid chromatography HPLC using an affinity chromatography column (trade name "POROS A20um Column", manufactured by Thermo Fisher Scientific Inc.). Phosphate buffered saline (pH of 7.4) was used as the solvent, and a 20 mmol/L phosphate buffer solution (pH of 2.8, sodium chloride concentration of 150 mmol/L) was used as the eluent.

### (Measurement of conductivity)

The conductivity of the culture supernatant or the desalting treatment liquid of the culture supernatant was measured by a desktop pH meter F-74 conductivity (electrical conductivity) electrode: 3552-10D (manufactured by HORIBA

### [Example 1]

A commercially available synthetic adsorbent (trade name "SEPABEADS 825L", manufactured by Mitsubishi Chemical Corporation, styrene-divinylbenzene copolymer subjected to porosification treatment) was crushed by a motar and washed with a 20 vol% ethanol aqueous solution using a filtration membrane (Merck & Co., Inc., pore diameter of 5 um), and adhesive moisture was removed by filtration, thereby obtaining a synthetic adsorbent (A).

The evaluation results of the obtained synthetic adsorbent (A) are listed in Table 2.

### [Example 2]

A commercially available synthetic adsorbent (trade name "SEPABEADS 825L", manufactured by Mitsubishi Chemical Corporation, styrene-divinylbenzene copolymer subjected to porosification treatment) was crushed by a motar, size-adjusted by a 20 um sieve, and washed with a 20 vol% ethanol aqueous solution using a filtration membrane (Merck & Co., Inc., pore diameter of 5 pm), and adhesive moisture was removed by filtration, thereby obtaining a synthetic adsorbent (B).

The evaluation results of the obtained synthetic adsorbent (B) are listed in Table 2.

### [Example 3]

A commercially available synthetic adsorbent (trade name "SEPABEADS 825L", manufactured by Mitsubishi Chemical Corporation, styrene-divinylbenzene copolymer subjected to porosification treatment) was crushed by a motar, size-adjusted by a 63 um sieve, and washed with a 20 vol% ethanol aqueous solution using a filtration membrane (Merck & Co., Inc., pore diameter of 5 pm), and adhesive moisture was removed by filtration, thereby obtaining a synthetic adsorbent (C).

The evaluation results of the obtained synthetic adsorbent (C) are listed in Table 2.

### [Comparative Example 1]

A styrene-divinylbenzene copolymer subjected to a porosification treatment was washed with a 20 vol% ethanol aqueous solution using a filtration membrane (Merck & Co., Inc., pore diameter of 5 um) without being crushed, and adhesive moisture was removed by filtration, thereby obtaining a synthetic adsorbent (G) that had not been crushed.

The evaluation results of the obtained synthetic adsorbent (G) are listed in Tables 2 and 3.

**[Table 2]**

| | Type of synthetic adsorbent | Particle size distribution [µm] | | | Differential pore volume [mL/g] | Corrected roundness | Crushing strength [gf/particles] | HCP concentration [ng/mL] | LRV | Antibody recovery rate [%] |
|---|---|---|---|---|---|---|---|---|---|---|
| | | d (0.1) | d (0.5) | d (09) | | | | | | |
| Culture solution | - | - | - | - | - | - | - | 512000 | - | - |
| Example 1 | Synthetic adsorbent (A) | 4 | 27 | 101 | 0.348 | 0.722 | 316 | 3950 | 2.5 | 78 |
| Example 2 | Synthetic adsorbent (B) | 38 | 99 | 256 | 0.383 | 0.764 | 316 | 5770 | 2.2 | 88 |
| Example 3 | Synthetic adsorbent (C) | 87 | 139 | 221 | 0.112 | 0.675 | 316 | 32700 | 1.3 | 93 |
| Comparative Example 1 | Synthetic adsorbent (G) | 86 | 118 | 162 | 0.044 | 0.052 | - | 374000 | 0.2 | 100 |

As listed in Table 2, the synthetic adsorbents (A) to (C) produced in Examples 1 to 3, in which the differential pore volume was greater than 0.05 mL/g, were excellent in HCP concentration, LRV, and antibody recovery rate as compared with the synthetic adsorbent (G) of Comparative Example 1, in which the differential pore volume was 0.05 mL/g or less. Particularly, the synthetic adsorbent (A) of Example 1 with a small volume average particle diameter had a low HCP concentration and excellent performance of removing impurities. Further, the synthetic adsorbent (C) of Example 3 with a large volume average particle diameter had a high antibody recovery rate and enabled efficient production of antibodies.

Further, the relationship between the measured pressure values of the differential pore volumes and the differential pore volumes (increased pore volumes) of the synthetic adsorbent (A), the synthetic adsorbent (B), the synthetic adsorbent (C), and the synthetic adsorbent (G) are shown in Fig. 1. As shown in Fig. 1, it can be seen that the maximum values of the differential pore volumes of the synthetic adsorbent (A), the synthetic adsorbent (B), and the synthetic adsorbent (C) are significantly greater than that of the synthetic adsorbent (G).

### [Example 4]

A commercially available synthetic adsorbent (trade name "SEPABEADS 850L", manufactured by Mitsubishi Chemical Corporation, styrene-divinylbenzene copolymer subjected to porosification treatment, pore radius of 5 nm) was crushed by a motar and washed with a 20 vol% ethanol aqueous solution using a filtration membrane (Merck & Co., Inc., pore diameter of 5 pm), and adhesive moisture was removed by filtration, thereby obtaining a synthetic adsorbent (D).

The evaluation results of the obtained synthetic adsorbent (D) are listed in Table 3.

### [Example 5]

A commercially available synthetic adsorbent (trade name "SEPABEADS 825L", manufactured by Mitsubishi Chemical Corporation, styrene-divinylbenzene copolymer subjected to porosification treatment, pore radius of 7 nm) was crushed by a motar and washed with a 20 vol% ethanol aqueous solution using a filtration membrane (Merck & Co., Inc., pore diameter of 5 um), and adhesive moisture was removed by filtration, thereby obtaining a synthetic adsorbent (E).

The evaluation results of the obtained synthetic adsorbent (E) are listed in Table 3.

### [Example 6]

A commercially available synthetic adsorbent (trade name "SEPABEADS HP21", manufactured by Mitsubishi Chemical Corporation, styrene-divinylbenzene copolymer subjected to porosification treatment, pore radius of 11 nm) was crushed by a motar and washed with a 20 vol% ethanol aqueous solution using a filtration membrane (Merck & Co., Inc., pore diameter of 5 um), and adhesive moisture was removed by filtration, thereby obtaining a synthetic adsorbent (F).

The evaluation results of the obtained synthetic adsorbent (F) are listed in Table 3.

**[Table 3]**

| | Type of synthetic adsorbent | Volume average particle diameter [µm] | Pore radius [nm] | Differential pore volume [mL/g] | Corrected roundness | Crushing strength [gf/particles] | HCP concentration [ng/mL] | LRV | Antibody recovery rate [%] |
|---|---|---|---|---|---|---|---|---|---|
| Culture solution | - | - | - | - | - | - | 512000 | - | - |
| Example 4 | Synthetic adsorbent (D) | 72 | 5 | 0.100 | 0.635 | 546 | 63500 | 0.8 | 96 |
| Example 5 | Synthetic adsorbent (E) | 55 | 7 | 0.131 | 0.619 | 316 | 4100 | 2.0 | 91 |
| Example 6 | Synthetic adsorbent (F) | 112 | 11 | 0.141 | 0.627 | 391 | 1700 | 2.5 | 62 |
| Comparative Example 1 | Synthetic adsorbent (G) | 118 | 7 | 0.044 | 0.052 | - | 374000 | 0.2 | 100 |

As listed in Table 3, the synthetic adsorbents (D) to (F) produced in Examples 4 to 6 were excellent in HCP concentration, LRV, and antibody recovery rate as compared with the synthetic adsorbent (G) produced in Comparative Example 1, which had not been crushed. Particularly, the synthetic adsorbent (F) of Example 6 with a large pore radius had a low HCP concentration and excellent performance of removing impurities. Further, the synthetic adsorbent (D) of Example 4 with a small pore radius had a large antibody recovery rate and enables efficient production of antibodies.

### [Example 7]

A commercially available synthetic adsorbent (trade name "SEPABEADS 825L", manufactured by Mitsubishi Chemical Corporation, styrene-divinylbenzene copolymer subjected to porosification treatment) was crushed by a motar, immersed in a 20 vol% ethanol aqueous solution, and subjected to suction filtration using a filtration membrane (Merck & Co., Inc., pore diameter of 5 pm), thereby obtaining a synthetic adsorbent (H). The maximum value of the measured differential pore volume of the synthetic adsorbent (H) was 0.131 mL/g.

As a culture solution of monoclonal antibodies (immunoglobulin G, molecular weight: 150000), a culture solution supernatant obtained by causing Chinese hamster ovary-derived cells (CHO cells) to produce antibodies, removing the CHO cells, and being clarified was used. The pH of the culture solution was adjusted from 7.4 to 4.5 by using a 5 mol/L acetic acid aqueous solution.

Next, 1.5 g of the synthetic adsorbent (H) and 30 mL of the prepared culture solution were weighed to a container, and the mixture was shaken and mixed at 25°C for 8 hours. Thereafter, the synthetic adsorbent was removed by centrifugation and a filtration membrane (Merck & Co., Inc., pore diameter of 0.22 pm), thereby obtaining a batch type treatment liquid of the synthetic adsorbent.

The pH of the batch type treatment liquid of the obtained synthetic adsorbent was adjusted to 3.5 with 1 mol/L hydrochloric acid, and the treatment liquid was allowed to stand at 25°C for 2 hours. Thereafter, the pH of the treatment liquid was adjusted to 5.0 with a 1 mol/L Tris solution, and the treatment liquid was filtered, thereby obtaining a virus inactivation solution.

The obtained virus inactivation solution was diluted to 4 times with MilliQ water, and the diluted virus inactivation solution was added to an anion exchange chromatography column(trade name "ChromSpeed Q103", manufactured by Mitsubishi Chemical Corporation) equilibrated with an equilibrium buffer solution of a 20 mmol/L tris-hydrochloric acid buffer solution (pH of 8.5). After completion of the addition, 5 column volumes of the equilibrium buffer solution was allowed to pass through the column. The column non-adsorbed fraction was used as a ChromSpeed Q103 eluent.

The pH of the obtained ChromSpeed Q103 was adjusted to 4.5 with 5 mol/L acetic acid, and the prepared eluent was added to an anion exchange chromatography column(trade name "ChromSpeed S103", manufactured by Mitsubishi Chemical Corporation) equilibrated with a 20 mmol/L acetic acid buffer solution (pH of 4.5). After completion of the addition, the adsorbed fraction was eluted into an eluent (20 mmol/L acetic acid buffer solution in which 500 mmol/L of sodium chloride was dissolved, pH of 4.5), and the eluent was used as a three-stage purified sample.

The HCP concentrations and the antibody concentrations of the culture solution supernatant, the obtained treatment liquid of the synthetic adsorbent, and the three-stage purified treatment liquid were measured by the following method.

The concentration of host cell-derived proteins (HCP concentration) [ng/mL] was measured by the ELISA method using a kit (kit name "ELISA Kit F550 (CHO Host Cell Protein, 3rd Generation).

The antibody purity [HCP/Mab ppm] was measured by high performance chromatography HPLC using an affinity chromatography column (trade name "POROS A 20 um Column", manufactured by Thermo Fisher Scientific Inc.).

The recovery rate of antibodies [%] was measured by high performance chromatography HPLC using an affinity chromatography column (trade name "POROS A 20 um Column", manufactured by Thermo Fisher Scientific Inc.). Phosphate buffered saline (pH of 7.4) was used as the solvent, and a 20 mmol/L phosphate buffer solution (pH of 2.8, sodium chloride concentration of 150 mmol/L) was used as the eluent.

### [Comparative Example 2]

As a culture solution of monoclonal antibodies (immunoglobulin G, molecular weight: 150000), a culture solution supernatant obtained by causing Chinese hamster ovary-derived cells (CHO cells) to produce antibodies, removing the CHO cells, and being clarified was used. The pH of the culture solution was adjusted from 7.4 to 4.5 by using a 5 mol/L acetic acid aqueous solution.

30 mL of the prepared culture solution was added to an affinity chromatography column (trade name "MabSelect SuRe tm LX", manufactured by GE Healthcare Life Science, column containing adsorbent (I) as an affinity adsorbent) and washed with 20 mmol/L phosphate buffered saline in a state of being adsorbed. The solution was desorbed with 100 mmol/L acetic acid in which 150 mmol/L of sodium chloride were dissolved, thereby obtaining a batch type treatment liquid of the adsorbent.

The batch type treatment liquid of the obtained adsorbent was diluted to 10 times with MilliQ water, the pH of the treatment liquid was adjusted to 3.5 with 1 mol/L hydrochloric acid, and the treatment liquid was allowed to stand at 25°C for 2 hours. Thereafter, the pH thereof was adjusted to 5.0 with a 1 mol/L Tris solution, and the treatment liquid was filtered, thereby obtaining a virus inactivation solution.

The obtained virus inactivation solution was added to an anion exchange chromatography column (trade name "ChromSpeed Q103", manufactured by Mitsubishi Chemical Corporation) equilibrated with an equilibrium buffer solution of a 20 mmol/L tris-hydrochloric acid buffer solution (pH of 8.5). After completion of the addition, 5 column volumes of the equilibrium buffer solution was allowed to pass through the column. The column non-adsorbed fraction was used as a ChromSpeed Q103 eluent.

The eluent obtained from the obtained ChromSpeed Q103 eluent with a cation exchange chromatography column by the same operation as in Example 7 was used as a three-stage purified sample, and the culture solution supernatant, the synthetic adsorbent treatment liquid, and the three-stage purified treatment liquid were analyzed in the same manner as in Example 7.

The results of Example 7 and Comparative Example 2 are listed in Table 4.

**[Table 4]**

| | Type of synthetic adsorbent | Type of liquid | HCP concentration [ng/mL] | Antibody purity [HCP/Mab ppm] | Cumulative antibody recovery rate [%] |
|---|---|---|---|---|---|
| Example 7 | Synthetic adsorbent (H) | Culture solution supernatant | 624732 | 277137 | 100 |
| | | Treatment liquid | 927 | 523 | 82 |
| | | Three-stage purification | 34 | 8 | 70 |
| Comparative Example 2 | Synthetic adsorbent (I) | Culture solution supernatant | 505852 | 249895 | 100 |
| | | Treatment liquid | 340 | 177 | 84 |
| | | Three-stage purification | 30 | 9 | 70 |

As listed in Table 4, the synthetic adsorbent (H) produced in Example 7 was crudely purified at a three-digit ppm level of the antibody purify and had a recovery rate of 80% or greater even though adsorption and desorption of antibodies and the washing step were not present in the first-stage adsorbent treatment as in a case of the adsorbent (I), as compared with the adsorbent (I) as the affinity adsorbent used in Comparative Example 2. The final sample of the three-stage purification had the same HCP removal rate and the same antibody recovery rate as those obtained by performing a step of using the adsorbent (I) as an affinity adsorbent.

### [Example 8]

### (preparation of culture solution supernatant)

As a culture solution of monoclonal antibodies (immunoglobulin G, molecular weight: 150000), a culture solution supernatant 1 (antibody concentration of 2.4 mg/mL, conductivity of 20 mS/cm) obtained by causing Chinese hamster ovary-derived cells (CHO cells) to produce antibodies, removing the CHO cells, and being clarified was used.

### (Desalting treatment)

0.4 g of an anion exchange resin (DIAION (registered trademark), "UBA 1200H", manufactured by Mitsubishi Chemical Corporation) and 0.4 g of a cation exchange resin (DIAION (registered trademark), "UBK 10H", manufactured by Mitsubishi Chemical Corporation) were added to the 4 mL of the culture solution supernatant 1. Thereafter, the pH of the culture solution supernatant 1 was adjusted from 7.4 to 4.5 using a 0.1 mol/L sodium hydroxide aqueous solution.

Thereafter, the anion exchange resin and the cation exchange resin were removed by a filtration membrane (manufactured by Merck & Co., Inc., pore diameter of 0.22 um), thereby obtaining a desalting treatment liquid (conductivity of 3 mS/cm).

### (Purification using synthetic adsorbent)

Next, 0.3 g of the synthetic adsorbent (B) and 4 mL of the prepared desalting treatment liquid were weighed to a container and shaken at 25°C for 8 hours. Thereafter, the synthetic adsorbent (B) was removed by centrifugation and a filtration membrane (manufactured by Merck & Co., Inc., pore diameter of 0.22 pm), thereby obtaining a treatment liquid using the synthetic adsorbent (B). The evaluation results of the treatment liquid are listed in Table 5.

### [Example 9]

The evaluation results of the treatment liquid treated by performing the same step as in Example 8 except that the culture solution supernatant 2 in which the antibody concentration of the monoclonal antibody was adjusted to 15 mg/mL was used in place of the culture solution supernatant 1 are listed in Table 5.

### [Example 10]

The evaluation results of the treatment liquid treated by performing the same step as in Example 8 except that the desalting treatment was not performed are listed in Table 5.

### [Example 11]

The evaluation results of the treatment liquid treated by performing the same step as in Example 9 except that the desalting treatment was not performed are listed in Table 5.

**[Table 5]**

| | Type of synthetic adsorbent | pH | Antibody concentration [mg/mL] | Conductivity [mS/cm] | HCP concentration [ng/mL] | LRV | Antibody recovery rate [%] |
|---|---|---|---|---|---|---|---|
| Culture solution supernatant 1 | - | 7.4 | 2.4 | 20 | 508417 | - | - |
| Culture solution supernatant 2 | - | 7.4 | 15 | 20 | 394195 | - | - |
| Example 8 | Synthetic adsorbent (B) | 4.5 | 2.4 | 3 | 3412 | 2.4 | 89 |
| Example 9 | | 4.5 | 15 | 3 | 995 | 2.9 | 94 |
| Example 10 | | 4.5 | 2.4 | 20 | 5352 | 2.2 | 86 |
| Example 11 | | 4.5 | 15 | 20 | 12009 | 1.9 | 88 |

As listed in Table 5, the HCP concentration, LRV, and the antibody recovery rate were excellent in Examples 8 and 9 in which the desalting treatment was performed, as compared with Examples 10 and 11 in which the process without the desalting treatment was carried out. Particularly, when the antibody concentration in the culture solution was high, the HCP concentration, LRV, and the antibody recovery rate were remarkably excellent.

### [Example 12]

The same treatment as in Example 7 was performed except that the desalting treatment described in Example 8 was performed on the culture solution supernatant, the synthetic adsorbent (B) was used in place of the synthetic adsorbent (H), and the virus inactivation solution was not diluted.

The evaluation results are listed in Table 6 collectively with the results of Example 7 and Comparative Example 2.

**[Table 6]**

| | Type of synthetic adsorbent | Type of liquid | HCP concentration [ng/mL] | Antibody purity [HCP/Mab ppm] | Cumulative antibody recovery rate [%] |
|---|---|---|---|---|---|
| Example 7 | Synthetic adsorbent (H) | Culture solution supernatant | 624732 | 277137 | 100 |
| | | Treatment liquid | 927 | 523 | 82 |
| | | Three-stage purification | 34 | 8 | 70 |
| Example 12 | Synthetic adsorbent (B) | Culture solution supernatant | 1031731 | 416021 | 100 |
| | | Desalting treatment liquid | 733652 | 380926 | 93 |
| | | Treatment liquid | 1043 | 727 | 90 |
| | | Three-stage purification | 81 | 23 | 81 |
| Comparative Example 2 | Synthetic adsorbent (I) | Culture solution supernatant | 505852 | 249895 | 100 |
| | | Treatment liquid | 340 | 177 | 84 |
| | | Three-stage purification | 30 | 9 | 70 |

As listed in Table 6, the desalting step (step (A)) was incorporated into the process in Example 12, and thus it was possible to omit the dilution to 4 times with MilliQ water before the anion exchange chromatography and the antibody recovery rate after the three-stage purification was 81%, which was relatively high value, as compared with Example 7. Further, the final sample of the three-stage purification had the same HCP removal rate as that obtained by performing a step of using the adsorbent (I) as an affinity adsorbent.

Although the present invention has been described in detail using specific embodiments, it will be apparent to those skilled in the art that various modifications can be made without departing from the spirit and scope of the present invention.

This application is based on Japanese Patent Application 2021-097149 filed on June 10, 2021, the entirety of which is incorporated by reference.

## Claims

1. A synthetic adsorbent,
wherein a maximum value of a differential pore volume (mL/g) under a pressure condition of 0.5 psia to 30.0 psia, which is measured by the following method, is greater than 0.05 mL/g,
<method of measuring differential pore volume (mL/g)>
1. a pressure in a sample container where the synthetic adsorbent that has been dried is placed is reduced to 10 Pa or less, mercury specified in JIS K 8572 is degassed by reducing the pressure to 10 Pa or less, and the sample container is filled with the mercury at a pressure of 0.5 psia,
2. a mercury intrusion amount when the pressure in the sample container filled with the mercury is stepwisely increased from 0.5 psia to 30.0 psia is measured, and
3. a differential pore volume (mL/g) is determined by dividing an amount of an increase in the mercury intrusion amount when a first-stage pressure calculated based on the mercury intrusion amount measured in the item 2 is increased, by a measured amount of the synthetic adsorbent.

2. The synthetic adsorbent according to claim 1,
wherein the synthetic adsorbent is a synthetic adsorbent for purifying an antibody.

3. The synthetic adsorbent according to claim 1 or 2, wherein the synthetic adsorbent has pores.

4. The synthetic adsorbent according to claim 3,
wherein the pores have a radius of 3 nm to 15 nm.

5. The synthetic adsorbent according to claim 1 or 2,
wherein the synthetic adsorbent is hydrophobic.

6. The synthetic adsorbent according to claim 1 or 2,
Wherein the synthetic adsorbent contains at least one selected from a styrene-based resin or an acrylic resin.

7. The synthetic adsorbent according to claim 1 or 2,
wherein the synthetic adsorbent has a volume average particle diameter of 1 um to 500 um.

8. The synthetic adsorbent according to claim 1 or 2,
wherein a corrected roundness value determined by the following method is greater than 0.10,
<method of measuring and calculating corrected roundness value>
1. the synthetic adsorbent is imaged by an optical microscope,
2. an approximate circle of the imaged synthetic adsorbent is created using image analysis software WinROOF2018,
3. the approximate circle is sandwiched between two concentric geometric circles (two concentric circles) that are concentric with the approximate circle in conformity with JIS B 0621,
4. a difference between a radius of the circle on an outer peripheral side and a radius of the circle on an inner peripheral side when a distance between the two concentric circles in the item 3 is the minimum is determined as the roundness,
5. the determined roundness is divided by a radius of the approximate circle, and
6. measurement and calculation in the items 1 to 5 are performed on 100 or more of circles, and an average value thereof is defined as the corrected roundness.

9. The synthetic adsorbent according to claim 1 or 2,
wherein a crushing strength is in a range of 100 gf/particles to 2000 gf/particles.

10. The synthetic adsorbent according to claim 2,
wherein the antibody is a monoclonal antibody.

11. The synthetic adsorbent according to claim 10,
wherein the monoclonal antibody has a molecular weight of 100000 or greater.

12. The synthetic adsorbent according to claim 10 or 11,
wherein the monoclonal antibody is immunoglobulin G.

13. An antibody purification method comprising:
mixing a mixture that contains antibodies and impurities with the synthetic adsorbent according to claim 1 and filtering the mixture.

14. An antibody purification method comprising:
a step of causing a mixture that contains antibodies and impurities to pass through the synthetic adsorbent according to claim 1 and recovering non-adsorbed fractions containing antibodies, which have not been adsorbed on the synthetic adsorbent.

15. An antibody purification method comprising:
a step (i) which is a contact step of brining a mixed solution that contains antibodies and impurities into contact with the synthetic adsorbent according to claim 1; and
a step (ii) which is a separation step of separating the mixed solution and the synthetic adsorbent from each other after the step (i).

16. The antibody purification method according to claim 15, further comprising:
a step (A) which is an ion exchange resin contact step of bringing a mixed solution that contains antibodies and impurities into contact with an anion exchange resin and/or a cation exchange resin,
wherein the step (A) is performed before the step (i).

17. The antibody purification method according to any one of claims 13 to 16,
wherein the impurities include a substance having a molecular weight of 50000 or less.

18. The antibody purification method according to any one of claims 13 to 16,
wherein the impurities include at least one selected from the group consisting of host cell-derived proteins, antibody-derived polymers, antibody-derived degradation products, and nucleic acids.

19. The antibody purification method according to any one of claims 13 to 16,
wherein the mixing of the mixture with the synthetic adsorbent, the passing of the mixture through the synthetic adsorbent, or the contacting of the mixed solution with the synthetic adsorbent are performed in a liquid having a pH of 3 to 8.

20. An antibody production method comprising:
the antibody purification method according to any one of claims 13 to 16.
